# EUROPÄISCHE PATENTSCHRIFT

(11) **EP 1 613 226 B1**
(45) Veröffentlichungstag und Bekanntmachung des Hinweises auf die Patenterteilung: **23.05.2007**
(21) Anmeldenummer: 04707936.3
(22) Anmeldetag: 04.02.2004
(51) Int. Cl.: A61B 17/72, A61F 2/30

(54) **VORRICHTUNG ZUM VERLÄNGERN VON KNOCHEN ODER KNOCHENTEILEN**
DEVICE FOR LENGTHENING BONES OR BONE PARTS
DISPOSITIF PERMETTANT D'ALLONGER DES OS OU DES PARTIES D'OS

(30) Priorität: 16.04.2003 DE 10317776
(43) Veröffentlichungstag der Anmeldung: 11.01.2006
(73) Patentinhaber: Wittenstein AG, 97999 Igersheim (DE)
(72) Erfinder: STAUCH, Roman, 97959 Assamstadt (DE)
(74) Vertreter: Weiss, Peter
(86) Internationale Anmeldenummer: PCT/EP2004/001005
(87) Internationale Veröffentlichungsnummer: WO 2004/091414

(56) Entgegenhaltungen:
- EP-A- 1 033 112
- DE-A- 19 708 279
- DE-C- 19 527 822
- US-A- 5 356 411
- US-A- 5 704 938
- US-A- 5 720 746

## Beschreibung

Die Erfindung betrifft eine Vorrichtung zum Verlängern von Knochen oder Knochenteilen für den Segmenttransport mit zumindest zwei gegeneinander bewegbaren Elementen, wobei zumindest ein Riegelelement in oder entlang eines Führungselementes axial bewegbar ist und das zumindest eine Riegelelement mittels zumindest einer Antriebseinrichtung in oder entlang des Führungselementes axial bewegbar angetrieben ist.

Derartige Vorrichtungen sind beispielsweise als Marknägel bekannt, die aus zwei gegeneinander bewegbaren Elementen, insbesondere Hülsen bestehen, die beispielsweise mittels elektrischen oder hydraulischen Antrieben auseinanderbewegbar sind, um einen Knochen zu verlängern oder ein Segment zu transportieren.

Nachteilig bei den herkömmlichen Vorrichtungen ist, dass diese einen äusserst geringen Hub für den Segmenttransport aufweisen und eine unerwünscht grosse Einbaulänge besitzen. Zudem sind diese aufwendig zu reinigen und zu betreiben.

Die DE 197 08 279 A1 offenbart ein Distraktionssystem für Röhrenknochen für den Segmenttransport, wobei innerhalb des Marknagels eine Hülse zur Aufnahme eines Befestigungselementes zum Festlegen eines Knochensegmentes gebildet ist, in welche eine antreibbare Gewindespindel eingreift. Hierdurch ist die Länge des Segmenttransportes bei beschränkter Querschnittsgrösse eingeschränkt.

Die DE 195 27 822 Cl beschreibt einen intrakorporalen Knochenmarknagel, welcher aus zwei gegeneinander bewegbaren Teilen gebildet ist.

Eine ähnliche Vorrichtung zum Verlängern von Knochen und Gewebe beschreibt die US 5,356,411, bei welcher zwei Teile einer Distraktionsvorrichtung mittels einer Spindel auseinanderbewegt werden.

Aus der US 5, 720, 746 ist eine mechanische Vorrichtung zum Verschieben zweier Elemente gegeneinander beschrieben, wobei aus dem einen Element eine zweites Element herausbewegbar ist.

Die EP 1 033 112 A2 offenbart einen Marknagel zur Knochendistraktion, bei welchem in einem proximalen Endbereich eine Spule integriert ist, die der berührungslosen Speisung eines elektrisch betriebenen Motors dient.

Eine rein mechanisch betriebene Distraktionsvorrichtung geht aus der US 5,704,938 hervor, bei welcher über eine Spindel ein Teil einer Distraktionsvorrichtung gegenüber dem anderen Teil herausbewegbar ist.

Der vorliegenden Erfindung liegt die Aufgabe zugrunde, eine Vorrichtung zum Verlängern von Knochen oder Knochenteilen zu schaffen, welche die genannten Nachteile beseitigen und mit welchen ein sehr grosser Hub bei limitierter Einbaugrösse möglich ist.

Ferner soll eine derartige Vorrichtung leicht zu reinigen und zu desinfizieren, insbesondere auch zu demontieren sein. Zudem soll diese Vorrichtung exakt anzusteuern und zu regeln sein.

Zur Lösung dieser Aufgabe führt, dass die Antriebseinrichtung mit einem Motorelement mit ggf. nachgeschaltetem Getriebe und Steuereinheit, und an das Motorelement oder an das Getriebe anschliessendem Spindelelement gebildet ist, wobei auf dem Spindelelement der zumindest eine Riegel direkt aufsitzt, wobei das Spindelelement als Gewindestange ausgebildet ist, welche den zumindest einen Riegel durchgreift und mit diesem in Eingriff steht.

Bei der vorliegenden Erfindung hat sich als besonders vorteilhaft erwiesen, zumindest ein Riegelelement in ein Führungselement axial bewegbar einzusetzen oder dieses entlang eines Führungselementes zu führen. Bevorzugt ist die Ausführungsform, dass das zumindest eine Riegelelement in einen Führungsschlitz eines Führungselementes eingesetzt ist und entlang dieses Fuhrungssehlitzes mittels eines Spindelelementes, einer Gewindestange od. dgl. axial hin und her bewegbar ist. Das Spindelelement wird mittels einer Antriebseinrichtung bzw. eines Motorelementes ggf. mit vorgeschalteten Getriebe angetrieben und bewegt exakt und genau den Riegel axial im Führungsschlitz hin und her. Der Riegel, der ggf. das Führungselement nach aussen übergreift, dient der Aufnahme eines Knochensegmentes, welches von einem Knochen oder Knochenteil abgetrennt wurde. Vorzugsweise wird sehr langsam, beispielsweise 0,5 mm bis 1,5 mm, vorzugsweise 1 mm je Tag das von einer Trennstelle vom Knochen oder Knochenteil abgetrennte Knochensegment, welches am Riegelelement festgelegt ist, von diesem distrahiert bzw. zum gegenüberliegenden Knochenteil oder Knochen bewegt, so dass auf diese Weise ein Segmenttransport und ein Wiederherstellen und Wachsen des Knochens durch Osteosynthese gewährleistet wird.

Ferner soll im Rahmen der vorliegenden Erfindung auch liegen, dass beispielsweise an entsprechenden Nuten od. dgl. der Riegel eingreift und dort geführt ist und über ein Spindelelement, eine Gewindestange, ein Seilzug od. dgl. axial entlang des Führungselementes zum Segmenttransport des Knochensegmentes bewegt wird.

In einem weiteren Ausführungsbeispiel der vorliegenden Erfindung können auch zwei Riegelelemente dem Spindelelement aufsitzen und entlang des Führungselementes, vorzugsweise im Führungsschlitz geführt sein, so dass beispielsweise beidseitig von beiden gegenüberliegenden Knochenteilen ein abgetrenntes Knochensegment, am Riegelelement festgelegt wird und durch entsprechendes Betätigen der Antriebseinrichtung bzw. des Spindelelementes die Knochensegmente bzw. die Riegel aufeinander langsam zu bewegt werden, um einen vollständigen Knochen zu erzeugen.

Weitere Vorteile, Merkmale und Einzelheiten der Erfindung ergeben sich aus der nachfolgenden Beschreibung bevorzugter Ausführungsbeispiele sowie anhand der Zeichnungen; diese zeigen in
Figur la eine schematisch dargestellte Seitenansicht auf eine Antriebseinrichtung für eine Vorrichtung zum Verlängern von Knochen zum Einsetzen in ein Führungselement;
Figur 1b eine schematisch dargestellte Draufsicht des Führungselementes;
Figur 2 eine schematisch dargestellte Draufsicht auf die Vorrichtung zum Verlängern von Knochen, bestehend aus Führungselement mit eingesetzter Antriebseinrichtung;
Figur 3 eine schematisch dargestellte Seitenansicht der Vorrichtung gemäss Figur 2;
Figur 4 eine schematisch dargestellte Draufsicht der Vorrichtung gemäss Figur 2 in einer möglichen Gebrauchslage;
Figur 5 eine schematisch dargestellte Draufsicht auf ein weiteres Ausführungsbeispiel der Vorrichtung gemäss Figur 2;
Figur 6 eine schematisch dargestellte Draufsicht auf die Vorrichtung gemäss Figur 5 in einer möglichen Gebrauchslage.

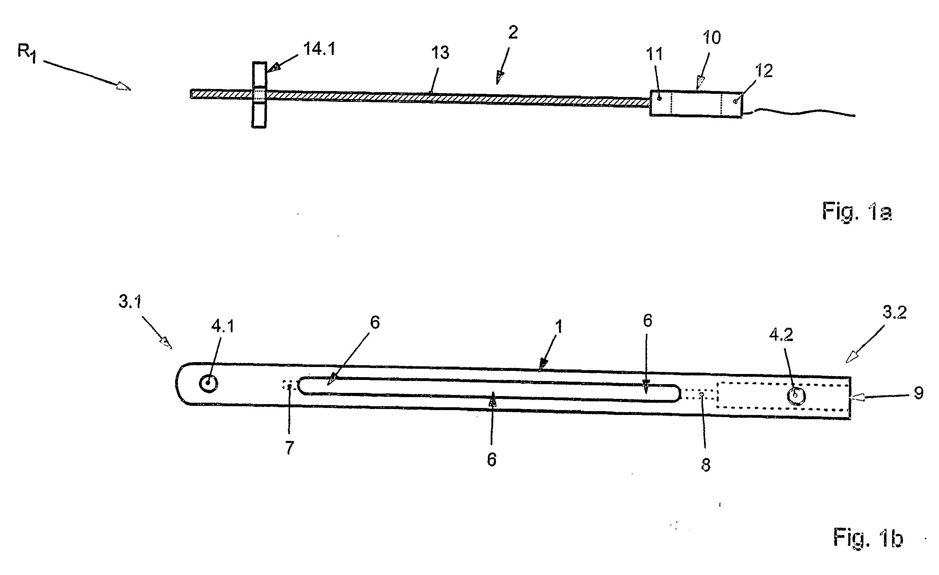

Gemäss den Figuren 1a und 1b weist eine erfindungsgemässe Vorrichtung R₁ zum Verlängern von Knochen oder Knochenteilen, insbesondere für den Segmenttransport ein Führungselement 1 und eine Antriebseinrichtung 2 auf. Das Führungselement 1 kann beispielsweise als Marknagel ausgebildet sein und ist jeweils im endseitigen Bereich 3.1, 3.2 mit Durchgangsöffnungen 4.1, 4.2 versehen, die dem Festlegen, insbesondere Verriegeln des Führungselementes 1 in oder mit einem Knochen 5 oder Knochenteil dienen. Hier nicht näher dargestellte Befestigungselemente legen das Führungselement 1 wiederlösbar im Knochen 5 fest.

In Axialrichtung ist ferner in das Führungselement, ein vorzugsweise durchgehender und länglicher Führungsschlitz 6 vorgesehen, welcher einends eine stirnseitige Lagerausnehmung 7, ausgeführt ggf. auch als Durchgangsbohrung, aufweist. Andernends schliesst stirnseitig an den Führungsschlitz 6 ein Durchgang 8 und daran vorzugsweise im endseitigen Bereich 3.2 eine Aufnahmeöffnung 9 an.

Die Antriebseinrichtung 2 besteht bevorzugt aus einem Motorelement 10 und ggf. vorgeschalteten Getriebe 11 und nachgeschalteter Steuereinheit 12.

An das Motorelement 10 und/oder das Getriebe 11 schliesst ein Spindelelement 13 antreibbar an, welchem zumindest ein Riegelelement 14.1 aufsitzt. Dabei kann das Spindelelement 13 beispielsweise als Gewindestange od. dgl. ausgebildet sein und durchgreift das Riegelelement 14.1 bzw. steht mit einem Innengewinde des Riegelelementes 14.1 in Eingriff.

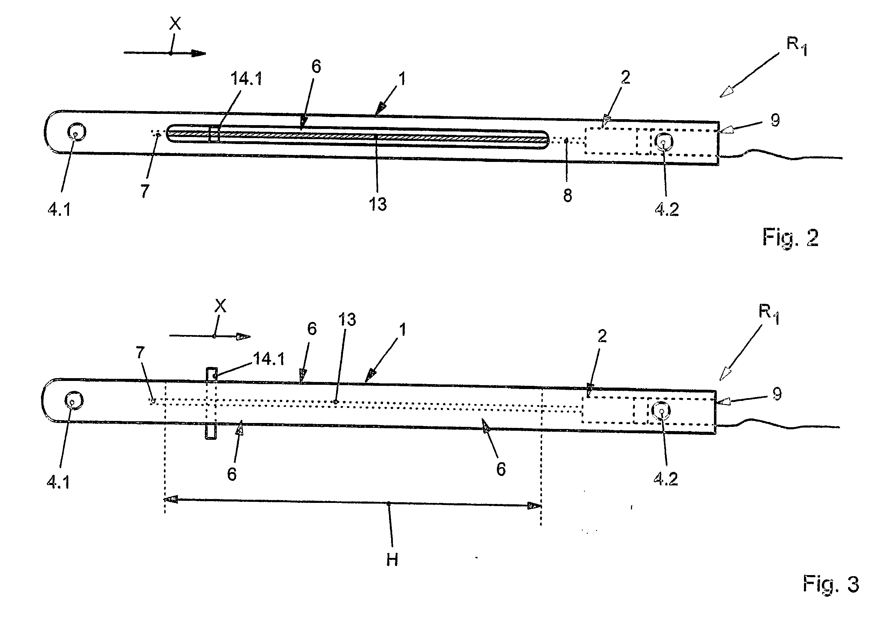

In der Draufsicht gemäss Figur 2 ist die Vorrichtung R₁ aufgezeigt, wobei die Antriebseinrichtung 2 in das Führungselement 1 eingesetzt ist. Dabei kann die Antriebseinrichtung 2 ohne Riegelelement 14.1 durch die Aufnahmeöffnung 9 des Führungselementes 1 in dieses eingeschoben werden, wobei das Riegelelement 14.1 durch den Führungsschlitz 6 eingeschoben wird und durch entsprechendes Antreiben des Spindelelementes 13 von diesem aufgenommen wird, wobei ein anschliessendes Lagern des endseitigen Bereiches des Spindelelementes 13 in der Lagerausnehmung 7 gewährleistet ist.

Die Antriebseinrichtung 2 lässt sich in dieser Lage, insbesondere das Motorelement 10 in der Aufnahmeöffnung 9 des Führungselementes 1 wiederlösbar festlegen, wobei über hier nicht dargestellte Verbindungskabel, induktive Adapter od. dgl. eine Einspeisung bzw. eine Antriebssteuerung der Antriebseinrichtung 2 erfolgt. In dieser Gebrauchslage lässt sich durch entsprechendes Betätigen des Motorelementes 10 bzw. Getriebes 11 das Spindelelement 13 rotativ antreiben, so dass auf diese Weise das Riegelelement 14.1 in oder entgegen einer dargestellten X-Richtung fahrbar bzw. bewegbar ist, wobei das Riegelelement 14.1 durch den Führungsschlitz 6 geführt ist und lediglich eine axiale Bewegung in dargestellter X-Richtung erfährt.

Dabei kann das Riegelelement 14.1, wie es insbesondere in der schematisch angedeuteten Seitenansicht aufgezeigt ist, entlang des Führungsschlitzes 6 je nach Antriebsrichtung des Spindelelementes 13 axial hin und her verfahren werden.

Die Länge des Führungsschlitzes 6 bestimmt einen Hub H, um welchen das Riegelelement 14.1 verfahren bzw. bewegt und angetrieben werden kann.

Die Funktionsweise der vorliegenden Erfindung ist folgende:

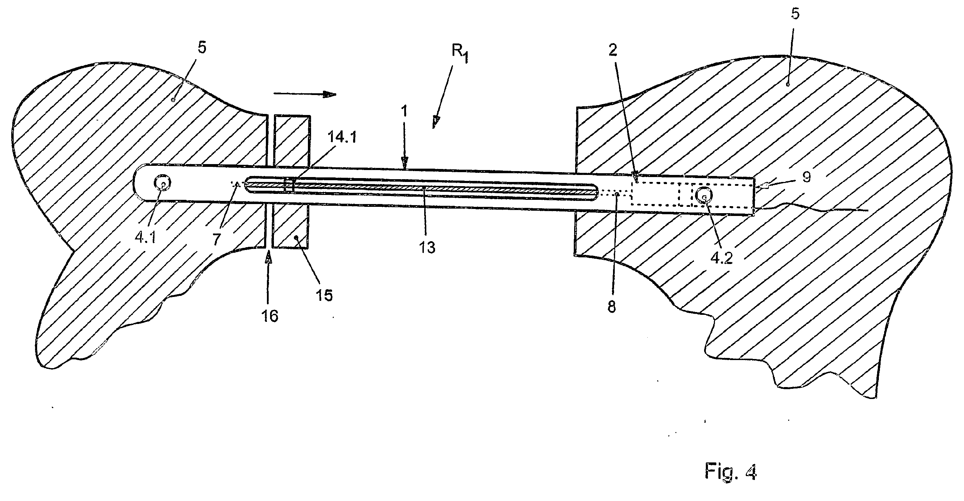

Die erfindungsgemässe Vorrichtung R₁ wird, wie es in Figur 4 angedeutet ist, beispielsweise in zwei endseitige Knochen 5 bzw. Knochenteile 5 eingesetzt, die mittels eines Knochensegmentes 15 durch Segmenttransport wieder zusammenwachsen sollen. Dabei wird von einem Knochen 5 ein Knochensegment 15 im Bereich einer hier angedeuteten Trennstelle 16 abgetrennt. Dann wird das Knochensegment 15 mit dem Riegel 14.1 verbunden oder greift in dieses ein oder wird mittels Befestigungselement dort festgelegt. Auch ist denkbar, dass das Riegelelement 14.1 zum Segmenttransport des Knochensegmentes 15 im Bereich der Trennstelle 16 angeordnet ist und das Knochensegment 15 hintergreift. Dann wird bspw. in zeitlich wählbaren Abständen mittels der Antriebseinrichtung 2 über das Motorelement 10 das Spindelelement 13 aktiviert und bewegt langsam, beispielsweise 0,5 mm bis 1,5 mm, vorzugsweise 1 mm pro Tag das Riegelelement 14.1 und damit das Knochensegment 15 zum gegenüberliegenden Knochen 5.

Auf diese Weise lässt sich der Knochen 5 wieder verlängern bzw. durch einen Segmenttransport vollständig wiederherstellen.

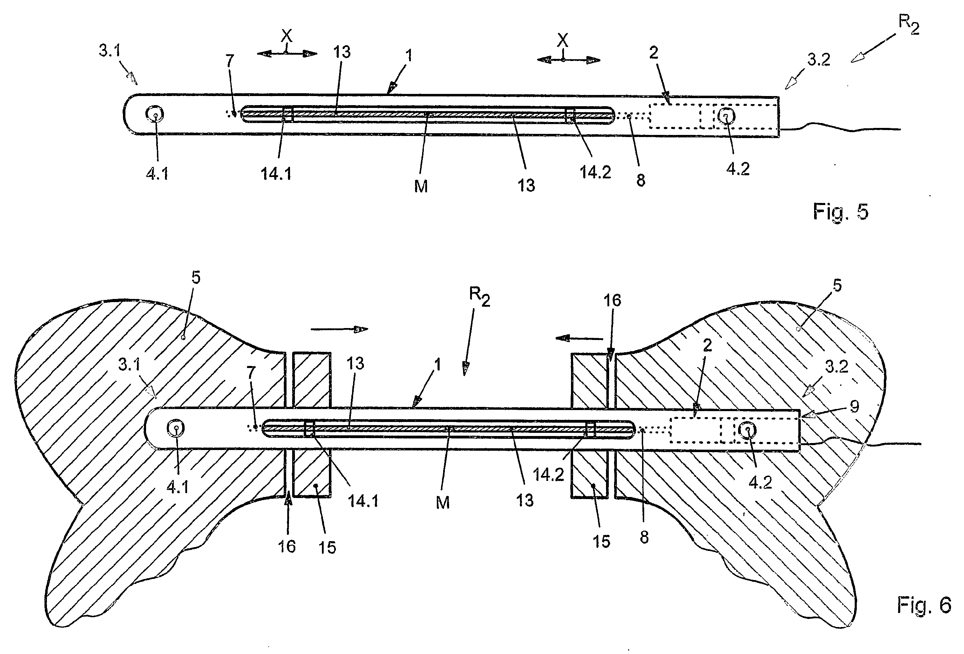

In einem weiteren Ausführungsbeispiel der vorliegenden Erfindung gemäss Figur 5 ist eine Vorrichtung R₂ aufgezeigt, die in etwa der o.g. Art entspricht. Unterschiedlich ist hier, dass dem Spindelelement 13 nahe dem endseitigen Bereich 3.1 das Riegelelement 14.1 und im Bereich des endseitigen Bereiches 3.2 ein weiteres Riegelelement 14.2 aufsitzen.

Vorzugsweise sind die Gewindegänge des Spindelelementes in der einen Hälfte als beispielsweise Rechts- und in der anderen Hälfte als beispielsweise Linksgewinde bzw. rechts- und linksgängig ausgeführt, so dass sich die Riegelelemente 14.1, 14.2, die vorzugsweise von einer Mitte M gleich weit beabstandet sind, sich gleichmässig gegeneinander oder auseinander durch die Antriebsbewegung des Spindelelementes 13 bewegen lassen.

Auf diese Weise lassen sich beispielsweise von jedem Knochen 5 bzw. Knochenteil Segmente 15 aufeinander zu bewegen, so dass beispielsweise bei einer Zustellbewegung von 1 mm je Tag sich ein Segmenttransport zweier Knochenteile wesentlich beschleunigen lässt. Dies soll ebenfalls im Rahmen der vorliegenden Erfindung liegen.

### Positionszahlenliste

| | | | | | |
|---|---|---|---|---|---|
| 1 | Führungselement | 34 | | 67 | |
| 2 | Antriebseinrichtung | 35 | | 68 | |
| 3 | endseitiger Bereich | 36 | | 69 | |
| 4 | Durchgangsöffnung | 37 | | 70 | |
| 5 | Knochen | 38 | | 71 | |
| 6 | Führungsschlitz | 39 | | 72 | |
| 7 | Lagerausnehmung | 40 | | 73 | |
| 8 | Durchgang | 41 | | 74 | |
| 9 | Aufnahmeöffnung | 42 | | 75 | |
| 10 | Motorelement | 43 | | 76 | |
| 11 | Getriebe | 44 | | 77 | |
| 12 | Steuereinheit | 45 | | 78 | |
| 13 | Spindelelement | 46 | | 79 | |
| 14 | Riegelelement | 47 | | | |
| 15 | Knochensegment | 48 | | | |
| 16 | Trennstelle | 49 | | R₁ | Vorrichtung |
| 17 | | 50 | | R₂ | Vorrichtung |
| 18 | | 51 | | | |
| 19 | | 52 | | X | Richtung |
| 20 | | 53 | | | |
| 21 | | 54 | | H | Hub |
| 22 | | 55 | | | |
| 23 | | 56 | | M | Mitte |
| 24 | | 57 | | | |
| 25 | | 58 | | | |
| 26 | | 59 | | | |
| 27 | | 60 | | | |
| 28 | | 61 | | | |
| 29 | | 62 | | | |
| 30 | | 63 | | | |
| 31 | | 64 | | | |
| 32 | | 65 | | | |
| 33 | | 66 | | | |

## Patentansprüche

1. Vorrichtung zum Verlängern von Knochen (5) oder Knochenteilen für den Segment transport mit zumindest zwei gegeneinander bewegbaren Elementen, wobei zumindest ein Riegelelement (14.1, 14.2) in oder entlang eines Führungselementes (1) axial bewegbar ist und das zumindest eine Riegelelement (14.1, 14.2) mittels zumindest einer Antriebseinrichtung (2) in oder entlang des Führungselementes (1) axial bewegbar angetrieben ist,
**dadurch gekennzeichnet,**
**dass** die Antriebseinrichtung (2) aus einem Motorelement (10) mit ggf. nachgeschaltetem Getriebe (11) und Steuereinheit (12), und an das Motorelement (10) oder an das Getriebe (11) anschliessendem Spindelelement (13) gebildet ist, wobei auf dem Spindelelement (13) der zumindest eine Riegel (14.1, 14.2) direkt aufsitzt, wobei das Spindelelement (13) als Gewindestange ausgebildet ist, welche den zumindest einen Riegel (14.1, 14.2) durchgreift und mit diesem in Eingriff steht.

2. Vorrichtung nach Anspruch 1, **dadurch gekennzeichnet, dass** das Führungselement (1) einen länglichen, vorzugsweise durchgehenden Führungsschlitz (6) aufweist.

3. Vorrichtung nach Anspruch. 1 oder 2, **dadurch gekennzeichnet, dass** das Führungselement (1) jeweils endseitig radiale Durchgangsöffnungen (4.1, 4.2) zu Durchführung und Einstecken von Befeatigungselementen zum Festlegen des Führungselementes (1) im Knochen (5) oder Knochenteile aufweist.

4. Vorrichtung nach wenigstens einem der Ansprüche 1 bis 3, **dadurch gekennzeichnet, dass** das Führungselement (1) einends eine hülsenartige Aufnahmeöffnung (9) zum Aufnehmen und Einstecken einer Antriebseinrichtung (2) aufweist.

5. Vorrichtung nach wenigstens einem der Ansprüche 1 bis 4, **dadurch gekennzeichnet, dass** durch radiales Verdrehen des Spindelelementes (13) oder der Gewindestange das in den Führungsschlitz (6) eingesetzte Riegelelement (14.1, 14.2) axial entlang des Führungselementes (1) hin und her bewegbar ist.

6. Vorrichtung nach wenigstens einem der Ansprüche 1 bis 5, **dadurch gekennzeichnet, dass** das Riegelelement (14.1, 14.2) querschnittlich rechteckartig oder rund ausgebildet ist und zumindest teilweise den Führungsschlitz (6) des Führungselementes (1) nach aussen übergreift.

7. Vorrichtung nach wenigstens einem der Ansprüche 1 bis 6, **dadurch gekennzeichnet, dass** über das Riegelelement (14) ein Knochenteil, insbesondere ein Knochensegment (15) durch Antreiben des Spindelelementes (13) mittels des Motorelementes (10) bewegbar ist, wobei zwischen einem Knochenteil und dem Knochensegment (15) eine Trennstelle (16) gebildet ist.

8. Vorrichtung nach wenigstens einem der Ansprüche 1 bis 7, **dadurch gekennzeichnet, dass** das Riegelelement (14.1, 14.2) zum Festlegen, insbesondere zum axialen Bewegen des Knochensegmentes (15) in dieses eingreift oder Befestigungselemente das Knochensegment (15) am Riegel (14.1, 14.2) lösbar festlegen.

9. Vorrichtung nach wenigstens einem der Ansprüche 4 bis 9, **dadurch gekennzeichnet, dass** die Antriebseinrichtung (2) in die Aufnahmeöffnung (9) axial einschiebbar ist und das Motorelement (10) verdrehfest im Führungselement (1), insbesondere im Bereich der Aufnahmeöffnung (9) eingesetzt ist.

10. Vorrichtung nach wenigstens einem der Ansprüche 1 bis 9, **dadurch gekennzeichnet, dass** einends des Führungsschlitzes (6) eine Lagerausnehmung (7) zur Lagerung des Spindelelementes (13) vorgesehen ist.

11. Vorrichtung nach wenigstens einem der Ansprüche 1 bis 10, **dadurch gekennzeichnet, dass** dem Spindelelement (13) zwei Riegelelemente (14.1, 14.2) aufsitzen, die bei Betätigen des Motorelementes (10) gegeneinander oder auseinander im Führungsschlitz (6) des Führungselementes (1) verfahrbar sind.

## Claims

1. Device for lengthening bones (5) or bone parts for segment transport comprising at least two mutually movable elements, wherein at least one locking element (14.1, 14.2) is movable axially in or along a guide element (1) and the at least one locking element (14.1, 14.2) may be set in axial motion in or along the guide element (1) by means of at least one drive device (2),
**characterized in**
**that** the drive device (2) is formed by a motor element (10) with optional downstream gear unit (11) and control unit (12), and by a spindle element (13) that adjoins the motor element (10) or the gear unit (11), wherein the at least one locking element (14.1, 14.2) is seated directly on the spindle element (13), wherein the spindle element (13) takes the form of a threaded rod that penetrates the at least one locking element (14.1, 14.2) and is in engagement therewith.

2. Device according to claim 1, **characterized in that** the guide element (1) has an elongate, preferably continuous guide slot (6).

3. Device according to claim 1 or 2, **characterized in that** the guide element (1) has at each end radial through-openings (4.1, 4.2) for the passage and insertion of fastening elements for fastening the guide element (1) in the bone (5) or bone parts.

4. Device according to at least one of claims 1 to 3, **characterized in that** the guide element (1) at one end has a sleeve-like receiving opening (9) for receiving and inserting a drive device (2).

5. Device according to at least one of claims 1 to 4, **characterized in that** by radial rotation of the spindle element (13) or of the threaded rod the locking element (14.1, 14.2) inserted into the guide slot (6) is movable axially back and forth along the guide element (1).

6. Device according to at least one of claims 1 to 5, **characterized in that** the locking element (14.1, 14.2) in cross section is of a rectangular or circular design and engages at least partially out beyond the guide slot (6) of the guide element (1).

7. Device according to at least one of claims 1 to 6, **characterized in that** via the locking element (14) a bone part, in particular a bone segment (15) is movable through driving of the spindle element (13) by means of the motor element (10), wherein a separation point (16) is formed between a bone part and the bone segment (15).

8. Device according to at least one of claims 1 to 7, **characterized in that** the locking element (14.1, 14.2) for fastening, in particular for axially moving the bone segment (15) engages therein or fastening elements fasten the bone segment (15) detachably to the locking element (14.1, 14.2).

9. Device according to at least one of claims 4 to 8, **characterized in that** the drive device (2) is insertable axially into the receiving opening (9) and the motor element (10) is inserted in a twist-proof manner in the guide element (1), in particular in the region of the receiving opening (9).

10. Device according to at least one of claims 1 to 9, **characterized in that** at one end of the guide slot (6) a bearing recess (7) is provided for supporting the spindle element (13).

11. Device according to at least one of claims 1 to 10, **characterized in that** two locking elements (14.1, 14.2) are seated on the spindle element (13) and upon actuation of the motor element (10) are displaceable towards or apart from one another in the guide slot (6) of the guide element (1).

## Revendications

1. Dispositif permettant d'allonger des os (5) ou parties d'os pour le transport de segments, avec au moins deux éléments déplaçables l'un par rapport à l'autre, au moins un élément de verrouillage (14.1, 14.2) étant déplaçable axialement dans ou le long d'un élément de guidage (1) et l'au moins un élément de verrou (14.1, 14.2) étant entraîné de manière déplaçable axialement au moyen d'au moins un dispositif d'entraînement (2) dans ou le long de l'élément de guidage (1),
**caractérisé par le fait que**
le dispositif d'entraînement (3) est constitué d'un élément de moteur (10) avec éventuellement un engrenage (11) connecté en aval et une unité de commande (12), et un élément de broche (13) raccordé à l'élément de moteur (10) ou à l'engrenage (11), sur l'élément de broche (13) se trouvant directement l'au moins un verrou (14.1, 14.2), l'élément de broche (13) étant réalisé sous forme de tige filetée qui traverse l'au moins un verrou (14.1, 14.2) et vient en prise avec ce dernier.

2. Dispositif selon la revendication 1, **caractérisé par le fait que** l'élément de guidage (1) présente une fente de guidage (6) allongée, de préférence continue.

3. Dispositif selon la revendication 1 ou 2, **caractérisé par le fait que** l'élément de guidage (1) présente à chaque extrémité des ouvertures de passage radiales (4.1, 4.5) destinées au passage et à l'introduction d'éléments de fixation destinés à fixer l'élément de guidage (1) dans l'os (5) ou les parties d'os.

4. Dispositif selon au moins l'une des revendications 1 à 3, **caractérisé par le fait que** l'élément de guidage (1) présente, à une extrémité, une ouverture de réception en forme de manchon (9) destinée à recevoir et à introduire un dispositif d'entraînement (2).

5. Dispositif selon au moins l'une des revendications 1 à 4, **caractérisé par le fait que** par la rotation radiale de l'élément de broche (13) ou de la tige filetée, l'élément de verrou (14.1, 14.2) placé dans la fente de guidage (6) peut être déplacé en va et vient le long de l'élément de guidage (1).

6. Dispositif selon au moins l'une des revendications 1 à 5, **caractérisé par le fait que** l'élément de verrou (14.1, 14,2) est réalisé de section rectangulaire ou ronde et vient en prise au moins partiellement vers l'extérieur par-dessus la fente de guidage (6) de l'élément de guidage (1).

7. Dispositif selon au moins l'une des revendications 1 à 6, **caractérisé par le fait que**, par l'intermédiaire de l'élément de verrou (14), une partie d'os, en particulier un segment d'os (15), peut être déplacé en entraînant l'élément de broche (13) au moyen de l'élément de moteur (10), entre une partie d'os et le segment d'os (15) étant formé un endroit de séparation (16).

8. Dispositif selon au moins l'une des revendications 1 à 7, **caractérisé par le fait que** l'élément de verrou (14.1, 14.2) s'engage, en vue de la fixation, en particulier du déplacement axial du segment d'os (15), dans ce dernier ou que des éléments de fixation fixent le segment d'os (15) de manière amovible au verrou (14,1, 14.2).

9. Dispositif selon au moins l'une des revendications 4 à 8, **caractérisé par le fait que** le dispositif d'entraînement (2) peut être introduit axialement dans l'ouverture de réception (5) et que l'élément de moteur (10) est placé de manière immobile en rotation dans l'élément de guidage (1), en particulier à l'endroit de l'ouverture de réception (9).

10. Dispositif selon au moins l'une des revendications 1 à 9, **caractérisé par le fait qu'**il est prévu, à une extrémité de la fente de guidage (6), un évidement de montage (7) destiné au montage de l'élément de broche (13).

11. Dispositif selon au moins l'une des revendications 1 à 10, **caractérisé par le fait que** sur l'élément de broche (13) se trouvent deux éléments de verrou (14.1, 14.2) qui, en cas d'actionnement de l'élément de moteur (10), peuvent être déplacés l'un par rapport à l'autre ou en s'éloignant l'un de l'autre dans la fente de guidage (6) de l'élément de guidage (1).
